# EUROPEAN PATENT APPLICATION

(11) **EP 4 379 377 A1**
(43) Date of publication of application: **05.06.2024**
(21) Application number: 22913436.6
(22) Date of filing: 26.08.2022
(51) Int. Cl.: G01N 33/531, G01N 33/543, G01N 33/68

(54) **MAGNETIC BEAD COATING, PREPARATION METHOD THEREFOR, AND TEST KIT**

(30) Priority: 27.12.2021 CN 202111615370
(71) Applicant: Shenzhen Yhlo Biotech Co., Ltd., Shenzhen, Guangdong 518116 (CN)
(72) Inventor: WANG, Xianle, Shenzhen, Guangdong 518116 (CN); QIAN, Ting, Shenzhen, Guangdong 518116 (CN); XIANG, Xufu, Shenzhen, Guangdong 518116 (CN); QIAN, Chungen, Shenzhen, Guangdong 518116 (CN)
(74) Representative: Michalski Hüttermann & Partner Patentanwälte mbB
(86) International application number: PCT/CN2022/115066
(87) International publication number: WO 2023/124154

(57) **Abstract**

A coated magnetic bead, a preparation method thereof, and a detection kit. The preparation method of the coated magnetic bead includes steps of: modifying a bovine serum albumin with a polyamine to produce a cationized bovine serum albumin; and coupling an active substance to a magnetic nanobead via the cationized bovine serum albumin to produce the coated magnetic bead. By producing the coated magnetic bead by the preparation method of the coated magnetic bead, the specificity of the coated magnetic bead and the immunogenicity of the active substance coated on the magnetic bead can be improved, thereby improving the specificity, the signal-to-noise ratio, the sensitivity, and the detection rate of the coated magnetic bead in the detection.

## Description

### TECHNICAL FIELD

The present application relates to the technical field of immunodetection, and in particular to a coated magnetic bead, a preparation method thereof, and a detection kit.

### BACKGROUND

The principle of immunodetection and immunodiagnosis on a chemiluminescent platform is as follows. An antigen or antibody is bound to a surface of a magnetic nanobead via physical adsorption or chemical crosslinking, while maintaining its immunological activity. During the detection, the test sample (containing an antigen or antibody to be detected) undergoes an immune reaction with the antigen or antibody on the surface of the magnetic bead and then forms a complex with an antigen or antibody labeled with a tracer (e.g., acridinium ester) in different steps, so that a quantitative or qualitative study can be realized based on luminescent signal.

A coated magnetic nanobead with an antigen or antibody coated on a surface of a magnetic nanobead is an important component of the luminescent immunoreagent. To ensure stable binding of the antigen or antibody to the surface of the magnetic particle, coupling by chemical cross-linking of the antigen or antibody is usually chosen. A small molecule peptide or a hapten molecule tends to have a low coating efficiency and cause poor bioactivity of the coated material when it is directly coated, because the molecular weight thereof is too small. Therefore, the small molecule peptide or the hapten molecule is often modified with a hydrophilic protein to increase the molecular weight thereof before the coupling reaction with the magnetic bead. Bovine serum albumin (BSA) is one of the commonly used modifying proteins and has been widely used in the modification of various small molecule peptides and hapten molecules due to its advantages of stable physicochemical properties, cheap and easy availability, large solubility, the ability to carry out the coupling reaction in both aqueous phase and organic phase (pyridine, DMF), simple operation, and so on.

However, the BSA-modified small molecule peptide or hapten molecule has a low coupling efficiency with the magnetic nanobead and causes a high amount of byproduct, which tends to result in a low specificity of the coated magnetic nanobead. In addition, the conventional BSA-modified small molecule peptide or hapten molecule tends to have a low immunogenicity, leading to a low signal-to-noise ratio of the luminescent immunoreagent and a failure to meet the performance requirements for the reagent.

### SUMMARY

In view of this, there is a need to provide a preparation method of a coated magnetic bead which can address the problem that the coated magnetic bead has a low specificity and that the BSA-modified small molecule peptide or hapten molecule has a low immunogenicity.

In addition, there is a need to provide a coated magnetic bead with good specificity and high immunogenicity and a detection kit including the coated magnetic bead.

A preparation method of a coated magnetic bead includes steps of:
modifying a bovine serum albumin with a polyamine to produce a cationized bovine serum albumin; and
coupling an active substance to a magnetic nanobead via the cationized bovine serum albumin to produce the coated magnetic bead.

In the above preparation method of the coated magnetic bead, the coated magnetic bead is prepared by modifying the bovine serum albumin into the cationized bovine serum albumin which is then coupled with the active substance and the magnetic bead, so that the bovine serum albumin has more amino groups and an isoelectric point thereof is increased from a range of 4.9 to 5.4 to a range of 9.5 to 11. As such, the bovine serum albumin can be coupled with the active substance more easily, with a higher coupling degree and less by-product of coupling, thereby improving the specificity of the resulting coated magnetic bead. In addition, as the isoelectric point of the bovine serum albumin is increased from a range of 4.9 to 5.4 to a range of 9.5 to 11, the immunogenicity of the active substance is significantly improved, and the ability of the active substance to bind with an antibody is enhanced, thereby increasing the signal-to-noise ratio in the immunodetection and allowing the detection kit including the above coated magnetic bead to have a higher sensitivity and a larger detection rate.

In one embodiment, the step of coupling the active substance to the magnetic nanobead via the cationized bovine serum albumin to produce the coated magnetic bead includes:
coupling the cationized bovine serum albumin to the active substance to produce a complex; and
coupling the complex to the magnetic nanobead to produce the coated magnetic bead.

In one embodiment, the polyamine is a diamine and/or a PEG-modified diamine.

In one embodiment, the diamine is selected from at least one of ethylenediamine, 1-3 diaminopropane, diaminodipropylamine, or hexanediamine.

In one embodiment, the step of modifying the bovine serum albumin with the polyamine to produce the cationized bovine serum albumin includes:
mixing the polyamine, the bovine serum albumin, and an activator and subjecting them to a dehydration condensation reaction to produce the cationized bovine serum albumin.

In one embodiment, a molar ratio of the bovine serum albumin to the polyamine is 1:5 to 1:20.

In one embodiment, the activator includes EDC and at least one of sulfo-NHS or NHS.

In one embodiment, a ratio of the complex to the magnetic nanobead is 40µg:1mg to 80µg:1mg.

A coated magnetic bead produced by the above preparation method of the coated magnetic bead is provided.

A detection kit including the above coated magnetic bead is provided.

### BRIEF DESCRIPTION OF THE DRAWINGS

FIG. 1 is a reaction formula of preparing a cBSA by modifying a BSA with a diamine according to an embodiment.
FIG. 2 is a flow chart of a reaction of coupling an active substance to the cBSA.

### DETAILED DESCRIPTION

In order to facilitate understanding of the present application, the present application will be described more comprehensively. The present application can be implemented in many different forms and therefore is not limited to the embodiments described herein. In contrast, the purpose of providing these embodiments is to thoroughly and comprehensively understand the disclosure of the present application.

The term "and/or" includes any and all combinations of one or more of the associated listed items.

Unless otherwise defined, all the technical and scientific terms used herein have the same meaning as commonly understood by the person skilled in the art to which the present application belongs. Herein, the terms used in the description of the present application are merely for the purpose of describing specific embodiments without limiting the present application. Herein, "BSA" is short for bovine serum albumin, and "cBSA" is short for cationized bovine serum albumin.

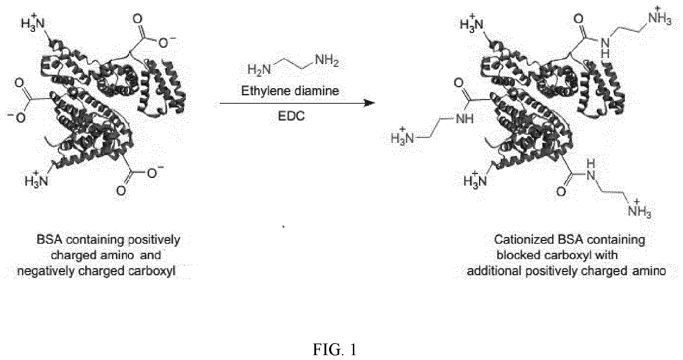

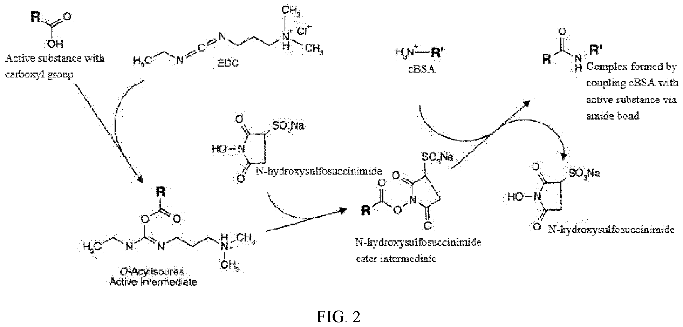

Referring to FIGs. 1 and 2, a preparation method of a coated magnetic bead is provided in an embodiment of the present application. The preparation method includes steps S100 and S200.

In step S100, a bovine serum albumin (BSA) is modified with a polyamine to produce a cationized bovine serum albumin (cBSA).

Specifically, the polyamine is a compound that contains two or more amino groups. The polyamine is used to provide the amino group so that a carboxyl group of the BSA is converted into an amino group after the modification with the polyamine.

In some embodiments, the polyamine includes a diamine and/or a PEG-modified diamine. The PEG-modified diamine refers to a diamine compound containing a hydrophilic chain of polyethylene glycol. In a reaction of modifying the BSA by the diamine, an amide bond is formed, which eliminates the negative potential of the carboxylate, while the terminal amine increases the potential of the positive charge, resulting in an increase in the isoelectric point of the modified BSA (i.e., cBSA) (pI is increased from the previous 4.9-5.4 to 9.5-11).

Optionally, the diamine is selected from at least one of ethylenediamine, 1-3 diaminopropane, diaminodipropylamine, or hexanediamine. Referring to FIG. 1, in one embodiment, the diamine is ethylenediamine. It should be understood that the polyamine is not limited to the above, nor the diamine limited to the above, and can be other substances capable of providing the amino group.

In some embodiments, the polyamine is the diamine, and a molar ratio of the BSA to the polyamine is 1:5 to 1:20. In an optional specific example, the molar ratio of the BSA to the polyamine is 1:5, 1:8, 1:10, 1:12, 1:15, 1:18, or 1:20. Further, the molar ratio of the BSA to the polyamine is 1:10 to 1:20. With the molar ratio of the BSA to the polyamine of 1: 10 to 1:20, the coupling efficiency of the BSA to the polyamine can be further improved and the generation of the by-product can be further reduced, thereby improving the specificity of the resulting coated magnetic bead for an antibody as an active substance, and further improving the signal-to-noise ratio of the detection. Further, the molar ratio of the BSA to the polyamine is 1:10 to 1:18.

In some embodiments, the step of modifying BSA with the polyamine includes mixing the polyamine, the BSA, and an activator and subjecting them to a dehydration condensation reaction.

The BSA contains carboxyl groups. Under the action of the activator, the amino group of the polyamine and the carboxyl group of the BSA are subjected to the dehydration condensation reaction to form the cBSA containing an amide bond.

In some embodiments, the activator includes at least one of N-hydroxysulfosuccinimide (sulfo-NHS) or N-hydroxysuccinimide (NHS), and 1-ethyl-(3-dimethylaminopropyl)carbodiimide (EDC), N,N-dicyclohexylcarbodiimide (DCC), or 1-cyclohexyl-3-(2-morpholinoethyl)carbodiimide (CMC). In one embodiment, at least one of sulfo-NHS or NHS, and EDC. It should be understood that in other embodiments, the activator is not limited to the above, and can be other substances that can cause the carboxyl group of BSA to form the amide bond with the amino group of the polyamine.

In some embodiments, the activator is employed at a concentration of 0.5 mg/mL to 2 mg/mL. In an optional specific example, the activator is employed at a concentration of 0.5 mg/mL, 1 mg/mL, 1.5 mg/mL, or 2 mg/mL. It should be noted that the concentration at which the activator is employed refers to the concentration of the activator at the time of addition of the activator to the reaction system.

In an optional specific example, the active substance is thyroxine (T4), the activator is consisted of EDC and sulfo-NHS, a solvent for dissolving the activator is ultrapure water, and a solvent for dissolving the active substance is DMSO. It should be understood that the solvents for dissolving the activator and the active substance can be selected according to the specific situation.

Specifically, the dehydration condensation reaction of the amino group of the polyamine with the carboxyl group of BSA is carried out in a buffer solution. Optionally, the buffer solution is MES, HEPES, MOPS, or PBS. Optionally, the buffer solution has a concentration of 50 mM to 100 mM.

In some embodiments, the pH of the system of the dehydration condensation reaction of the amino group of the polyamine with the carboxyl group of the BSA is 5 to 7. In an optional specific example, the pH of the system of the dehydration condensation reaction of the amino group of the polyamine with the carboxyl group of the BSA is 6.

It should be understood that after the dehydration condensation reaction of the amino group of the polyamine with the carboxyl group of the BSA, a step of purifying the cBSA in the reaction product is further included. Optionally, a method for purifying the cBSA includes, but is not limited to, at least one of desalination, ultrafiltration, or dialysis. In an optional specific example, ultrafiltration is employed to purify the cBSAin the reaction product.

In step S200, an active substance is coupled to the magnetic nanobead via the cationized bovine serum albumin to produce the coated magnetic bead.

Specifically, the active substance is a substance used in an immunodetection, which specifically binds to a substance to be detected in a test sample. After the active substance specifically binds to the substance to be detected, a marker which can specifically bind to the substance to be detected is utilized to calculate the amount of the substance to be detected in the test sample by detecting the amount of the marker, wherein the epitope at which the marker binds to the substance to be detected may be the same as or difference from the epitope at which the active substance binds to the substance to be detected; in case where the epitope at which the marker binds to the substance to be detected is the same as the epitope at which the active substance binds to the substance to be detected, the amount of the substance to be detected in the test sample is determined with the principle of competitive assay; in case where the epitope at which the marker binds to the substance to be detected is different from the epitope at which the active substance binds to the substance to be detected, the amount of the substance to be detected in the test sample is determined with the principle of sandwich assay. In some embodiments, the active substance is a small molecule peptide, a hapten, a protein, or a steroid. It should be understood that in other embodiments, the active substance can be other substances.

Referring to FIG. 2, in some embodiments, the step of coupling the active substance to the magnetic nanobead via the cBSAto produce the coated magnetic bead includes: coupling the cBSA to the active substance to produce a complex; and coupling the complex to the magnetic nanobead to produce the coated magnetic bead. The cBSA is coupled to the active substance to form the complex via a condensation reaction of the amino group of cBSA with the carboxyl group of the active substance. It should be understood that the condensation reaction of the amino group of the cBSA with the carboxyl group of the active substance is carried out in a buffer solution under the action of an activator. Optionally, the selection for the activator or the buffer solution for the condensation reaction of the amino group of the cBSA with the carboxyl group of the active substance is the same as the selection for the activator or the buffer solution for the dehydration condensation reaction of the amino group of the polyamine with the carboxyl group of the BSA. It should be noted that in FIG. 2, 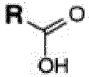 refers to the active substance containing the carboxyl group, H₃N*-R' refers to the cBSA, 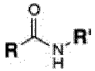 refers to the complex formed by coupling the cBSAto the active substance.

In some embodiments, the active substance contains one carboxyl group, and a molar ratio of the active substance to the cBSA is 1:1 to 10:1. In an optional specific example, the molar ratio of the active substance to the cBSA is 1:1, 5:1, 8:1, or 10:1. It should be understood that the amount of the cBSA can be adaptively adjusted when the active substance contains other number of carboxyl group .

It should be understood that after the condensation reaction of the cBSA with the active substance is finished, a step of purifying the complex in the reaction product is further included. Optionally, a method for purifying the complex in the reaction product includes, but is not limited to, at least one of desalination, ultrafiltration, or dialysis. In an optional specific example, ultrafiltration is employed to purify the complex in the reaction product.

In some embodiments, the magnetic nanobead is a carboxylated magnetic bead, and the complex is coupled to the carboxyl group of the magnetic nanobead via its amino group. It should be understood that in other embodiments, the complex can be coupled to the magnetic nanobead via other means such as a carboxyl group of the complex (e.g., the carboxyl groups of the BSA are not fully aminated or there is a carboxyl group on the polyamine). It should be understood that the type of the magnetic nanobead is not limited to the carboxylated magnetic bead, but can also be a tosylated magnetic bead or an aminated magnetic bead. The specific type of the magnetic nanobead can be selected according to the actual situation, as long as it can be coupled to the complex.

In some embodiments, a ratio of the complex to the magnetic nanobead is 40 µg:1 mg to 80 µg:1 mg. Further, the ratio of the complex to the magnetic nanobead is 50 µg:1 mg to 80 µg:1 mg.

Of course, after the complex is coupled to the magnetic nanobead, a step of blocking an unreacted active site is included. By blocking the unreacted active site, the substance to be detected is prevented from binding to other substances on the magnetic nanobead than the active substance. In some embodiments, a blocking agent is employed to block the active site. Optionally, the blocking agent is a protein (e.g., BSA), an antibody, or a serum. In one embodiment, the blocking agent is a protein blocking agent.

The above-described preparation method of the coated magnetic bead has at least the following advantages.
(1) The specificity of the coated magnetic bead is improved. By converting the carboxyl group of the BSA to the amino group, the amount of the amino group in the BSA molecule is significantly increased, which makes it easier for a single cBSA to couple with the active substance such as the small molecule peptide or the hapten molecule and allows a larger amount of the active substance to be coupled. In addition, since the surface of cBSA is extremely rich in amino groups and almost devoid of carboxyl groups, cBSA is more capable of directionally coupling to the carboxyl group of the active substance than BSA, which greatly improves the coupling efficiency, reduces the generation of the by-product, and improves the purity of the resulting complex formed by the active substance and the BSA, and thus improves the specificity of the coated magnetic bead. Moreover, due to the modification by the polyamine such as the diamine, the cBSA has the iso-electric point increased from about 5.0 to about 9.5-11 and thus is positively charged during the reaction, while the succinimide ester obtained by activating the active material using EDC and sulfo-NHS tends to be negatively charged and have increased reaction electrostatic charge attraction, which also significantly increases the reaction efficiency. In conclusion, the above-described magnetic bead coating improves the specificity of the coated magnetic bead by coupling the cBSA to the active substance. The significant increase in the specificity of the coated magnetic bead, which is obtained by coupling the complex formed by cBSA and the active substance to the magnetic bead, has been demonstrated upon testing.
(2) The sensitivity, the signal-to-noise ratio, and the detection rate in the detection are improved. The polyamine-modified BSA, as its isoelectric point is increased from the original 5.0 around to about 9.5-11, significantly improves the immunogenicity of the active substance and thus the ability of the active substance to bind to the antibody, thereby improving the signal-to-noise ratio of the reagent.

Based on the above, a coated magnetic bead is further provided in an embodiment of the present application. The coated magnetic bead is produced by the preparation method of the coated magnetic bead in any one of the above embodiments.

In some embodiments, the above-described coated magnetic bead includes a magnetic nanobead, a bovine serum albumin, and an active substance. Both the magnetic nanobead and the active substance are coupled to the bovine serum albumin. The carboxyl group of the bovine serum albumin has been modified by a polyamine. In one embodiment, the magnetic nanobead is coupled to the bovine serum albumin by forming an amide bond, and the bovine serum albumin is coupled to the active substance by forming an amide bond.

The above-described coated magnetic bead is produced by the above-described preparation method of the coated magnetic bead, and has been demonstrated to have good specificity, high signal-to-noise ratio, high sensitivity and high detection rate when applied to the detection.

Based on the above, a detection kit is further provided in an embodiment of the present application. The detection kit includes the coated magnetic bead in any one of above embodiments.

In some embodiments, the above-described detection kit further includes a labeled antibody. The labeled antibody includes an antibody that can specifically bind to a substance to be detected, and a signal substance labeled on the antibody. The amount of the substance to be detected in a test sample is reflected by detecting the signal substance labeled on the antibody. The signal substance refers to a substance that can provide a signal to be detected. The signal substance is generally labeled, via a reaction, on the antibody which can specifically bind to the substance to be detected.

In some embodiments, the signal substance is selected from at least one of a chromophore, a digoxigenin-labeled probe, an electron-dense substance, colloidal gold, or a signal- detectable enzyme. Optionally, the chromophore is selected from at least one of fluorescence, quantum dots, fluorescent microspheres, a luminescent compound, or a dye. In some embodiments, the chromophore is a luminescent compound such as acridinium ester or a derivative thereof, adamantane, luminol, isoluminol, etc. Optionally, the acridinium ester or a derivative thereof is selected from at least one of acridinium ester, acridinium sulfonamide, acridinium toluenesulfonamide, acridinium p-methylsulfonamide, or acridinium trifluoromethylsulfonamide. Optionally, the electron-dense substance is a radioactive molecule, for example 32P, 35S, or 125I. optionally, the enzyme producing the detectable signal is selected from one of horseradish peroxidase, alkaline phosphatase, beta-galactosidase, and glucose-6-phosphate dehydrogenase. It should be understood that an antibody or a signal substance for labeling an antibody is not particularly limited, and can be selected and adjusted according to the actual situation.

In some embodiments, the above-described detection kit further includes at least one of a buffer solution for dilution, an eluent, a protective protein solution, a luminescent substrate solution, and a reaction cup. Optionally, the protective protein in the protective protein solution is selected from at least one of neonatal bovine serum, ovalbumin, bovine serum albumin, or casein. The protective protein provides a good and stable reaction environment so that the antigen/antibody maintains its natural conformation.

In one embodiment, the above-described detection kit is used to detect a T4 antibody. The above-described detection kit includes a coated magnetic bead, wherein an active substance in the coated magnetic bead is T4.

The above-described detection kit includes the above-described coated magnetic bead. The above-described detection kit has better specificity, higher signal-to-noise ratio, better sensitivity, and higher detection rate than a conventional detection kit including a coated magnetic bead in which an active substance is coupled directly to the bovine serum albumin.

### Specific Examples

A detailed description will be given below in conjunction with specific examples. The following examples do not include components other than unavoidable impurities unless otherwise specified. The reagents and instruments used in the examples are conventional choices in the art unless otherwise specified. The experimental methods that do not specify specific conditions in the examples are implemented in accordance with conventional conditions, such as the conditions described in literature and books, or with the method recommended by the manufacturer.

### Example 1

### 1. Coupling of T4 (thyroxine) to BSA

(1) T4 (776.87 g/mol) was dissolved in DMSO to prepare a thyroid hormone T4 solution with a concentration of 5 mg/mL.
(2) BSA (65KD) was dissolved in a MES buffer at 0.1 M and pH 6 to prepare a BSA solution with a BSA concentration of 5 mg/mL. 7 mL of the BSA solution was taken out and added with 85 µL of the thyroid hormone T4 solution so that the molar ratio thereof in the reaction was about 1:1, to prepare a mixed solution.
(3) EDC and sulfo-NHS were respectively dissolved in water to 10 mg/mL and mixed in equal volumes (1: 1) to prepare an activator solution.
(4) 0.7 mL of the activator prepared in step (3) was added to the mixed solution prepared in step (2) (the concentration of the activator in the reaction was about 0.5 mg/mL) and placed in an incubator at 25°C to carry out the reaction for 3 h.
(5) The solution obtained after the reaction in step (4) was subjected to purification by ultrafiltration and the solid was stored in a phosphate buffer at 20 nM and pH 7.4 to prepare a solution containing BSA coupled to T4 (T4-BSA complex solution).

### 2. Preparation of T4-coated magnetic bead

(1) 10 mg of carboxylated magnetic beads was washed with a MES buffer at 0.1 M and pH 6 for three times and then added with 200µL of the MES buffer.
(2) EDC and sulfo-NHS were respectively dissolved in MES buffers so that the concentrations thereof were each 10 mg/mL, and they were mixed at 1:1. 200 µL of the mixture was added to the magnetic beads in (1) and subjected to a reaction for 30 min in an incubator at 25°C for use.
(3) A magnetic separation was performed and a supernatant was discarded. The magnetic beads were added with 1 mL of a MES buffer at 0.1 M and pH 6 and then with 0.4 mg of the T4-BSA complex (the concentration of the T4-BSA complex solution was measured with a UV protein concentration meter, and then such amount of the T4-BSA complex was calculated based on concentration × corresponding volume = mass), mixed uniformly, and placed in an incubator at 25°C and subjected to a reaction for 30 min under mixing.
(4) After the reaction was finished, a magnetic separation was performed. A phosphate buffer containing 0.5% BSA was added, and a blocking reaction was carried out under mixing in the incubator at 25°C for 2 h.
(5) After the blocking reaction was finished, a magnetic separation was performed. A certain volume was reached by using a phosphate buffer containing 0.5% BSA so that the concentration was 10 mg/mL (in terms of bare magnetic beads, the same below), the magnetic bead was stored at 4 °C for use.

### Example 2

### 1. Preparation of cBSA by coupling BSA to ethylenediamine

The reaction formula of coupling of BSA to ethylenediamine is shown in FIG. 1. The coupling of BSA to ethylenediamine includes, but is not limited to the following steps.
(1) BSA was dissolved in 0.1 M MES buffer to obtain a BSA solution with a concentration of 5 mg/mL.
(2) Ethylenediamine was dissolved in water and then the pH thereof was adjusted to 6.0 by a high concentration MES to prepare an ethylenediamine solution with a concentration of 5 mg/mL.
(3) 10 mL of the BSA solution prepared in step (1) was taken out and mixed with 92 µL of the ethylenediamine solution prepared in step (2) so that the molar ratio of BSA: ethylenediamine in a reaction after the mixing was 1: 10.
(4) EDC and sulfo-NHS were respectively dissolved in water to prepare solutions each with a concentration of 10 mg/mL, which were then mixed in equal volumes (1:1) to prepare an activator with a concentration of 0.5 mg/mL.
(5) 1 mL of the activator prepared in step (4) was added to step (3) and then subjected to a reaction for 3 h at room temperature of 25°C.
(6) After the reaction is finished, purification by ultrafiltration was performed to obtain purified cationized bovine serum albumin (cBSA), which was stored in a phosphate buffer at 20 mM and pH 7.4 for use.

### 2. Coupling of T4 to cBSA

(1) T4 was dissolved in DMSO to prepare a T4 solution with a concentration of 5 mg/mL. cBSAwas diluted in a MES buffer at 0.1 M and pH 6 to prepare a cBSA solution with a concentration of 5 mg/mL.
(2) 7 mL of the cBSA solution prepared in step (1) was added and mixed with 85µL of the T4 solution prepared in step (1) so that a molar ratio of cBSA to T4 in the reaction is 1:1.
(3) EDC and sulfo-NHS were respectively dissolved in water to prepare solutions each with a concentration of 10 mg/mL and then mixed in equal volumes. Then 0.7 mL of the mixture was added to the mixed solution of cBSA with T4 in the step (2) and placed in an incubator at 25°C to carry out a reaction for 3 h. After the purification by ultrafiltration, a T4-cBSA complex was prepared.

### 3. Preparation of T4-cBSA-coated magnetic bead

(1) 10mg of carboxylated magnetic beads was washed with a MES buffer at 0.1 M and pH 6 for three times and then added with 200µL of the MES buffer.
(2) EDC and sulfo-NHS were respectively dissolved in MES buffers so that concentrations thereof were each 10 mg/mL, and they were mixed at 1:1. Then 200 µL of the mixture was added to the magnetic beads in (1) and subjected to a reaction for 30 min in an incubator at 25°C for use.
(3) A magnetic separation was performed, and a supernatant was discarded. The magnetic beads were washed, then added with 1 mL of a MES buffer at 0.1 M and pH 6 and then with 0.4 mg of the T4-BSA complex, and mixed uniformly. The magnetic beads were placed in an incubator at 25°C and subjected to a reaction for 30 min under mixing.
(4) After the reaction was finished, a magnetic separation was performed, then a phosphate buffer containing 0.5% BSA was added, and a blocking reaction was carried out under mixing in an incubator at 25°C for 2 h.
(5) After the blocking reaction was finished, a magnetic separation was performed. A certain volume was reached by using a phosphate buffer containing 0.5% BSA so that the concentration of the magnetic beads was 10mg/mL, which was preserved at 4 °C for use.

### Example 3

The preparation of T4-cBSA coated magnetic bead in this example is substantially the same as Example 2, except that in this example, in the coupling of BSA to diethylamine, the molar ratio of BSA to diethylamine is 1:5.

### Example 4

The preparation of T4-cBSA coated magnetic bead in this example is substantially the same as Example 2, except that in this example, in the coupling of BSA to diethylamine, the molar ratio of BSA to diethylamine is 1:20.

### Comparative Example 1

(1) A commercially available T4-BSA complex was dissolved in water to 5mg/mL.
(2) 10mg of carboxylated magnetic beads was washed with a MES buffer at 0.1 M and pH 6 for three times and then added with 200 µL of the MES buffer.
(3) The activators EDC and sulfo-NHS were respectively dissolved in MES buffers so that concentrations thereof were each 10 mg/mL, and they were mixed at 1:1. Then 200 µL of the mixture was added to the magnetic beads in (1) and subjected to a reaction for 30 min in an incubator at 25°C for use.
(4) A magnetic separation was performed, and a supernatant was discarded. The magnetic beads were washed, then added with 1 mL of a MES buffer at 0.1 M and pH 6 and then with 0.4 mg of the T4-BSA complex of step (1), and mixed uniformly. The magnetic beads were placed in an incubator at 25°C and subjected to a reaction for 30 min under mixing.
(5) After the reaction was finished, a magnetic separation was performed, then a phosphate buffer containing 0.5% BSA was added, and a blocking reaction was carried out under mixing in incubator at 25°C for 2 h.
(6) After the blocking reaction was finished, a magnetic separation was performed. A certain volume was reached by using a phosphate buffer containing 0.5% BSA so that the concentration of the magnetic beads was 10 mg/mL, which was stored at 4 °C for use.

### Test

(1) Three kinds of coated magnetic beads of Example 1, Example 2, and Comparative Example 1 were diluted to a working concentration of 0.10 mg/mL. 100 negative samples and 15 gradient samples were collected for testing. The coated magnetic beads were reacted with samples and then formed complexes with acridinium esters labeled with T4 antibody. The luminescence values of the acridinium esters were measured, and the results of the tests are shown in Table 1 and Table 2.

**Table 1**

| Item | Example 1 | Example 2 | Comparative Example 1 |
|---|---|---|---|
| specificity | 92.0% | 98.0% | 91.0% |

In Table 1, the specificity refers to the negative detection rate. The specificity of 92% for Example 1 means that 92 negative samples were detected out of 100 negative samples. The specificity of 98% for Example 2 means that 98 negative samples were detected out of 100 negative samples. The specificity of 91% for Comparative Example 1 means that 91 negative samples were detected out of 100 negative samples.

**Table 2**

| Sample No. | Signal value | | |
|---|---|---|---|
| | Example 1 | Example 2 | Comparative Example 1 |
| 1 | 1958707 | 2573006 | 1894574 |
| 2 | 223331 | 424462 | 214261 |
| 3 | 152130 | 253123 | 149875 |
| 4 | 134515 | 202987 | 129741 |
| 5 | 110279 | 185699 | 10950 |
| 6 | 93125 | 172450 | 92524 |
| 7 | 79038 | 157829 | 73374 |
| 8 | 56284 | 89256 | 54129 |
| 9 | 37413 | 55263 | 38874 |
| 10 | 21451 | 25319 | 22013 |
| 11 | 15370 | 19896 | 14528 |
| 12 | 8836 | 10251 | 8527 |
| 13 | 5539 | 6780 | 5326 |
| 14 | 1580 | 1938 | 1409 |
| 15 | 833 | 783 | 798 |

As can be seen from Tables 1 and 2, the sensitivity (or signal-to-noise ratio) and the specificity of Example 2 were significantly better than those of Example 1 and Comparative Example 1, suggesting that as an antigen, the T4-cBSA obtained by modifying conventional BSA into cBSA before coupling results in better sensitivity and specificity than the directly coupled BSA. The differences in specificity and sensitivity between Example 1 and Comparative Example 1 are not significant, suggesting that the specificity and the sensitivity of directly coupled BSA as antigen is not due to test manipulation.

(2) Three kinds of coated magnetic beads of Example 2, Example 3, and Example 4 were diluted to a working concentration of 0.10 mg/mL. 8 gradient samples were collected for testing. The coated magnetic beads were reacted with samples and then formed complexes with acridinium esters labeled with T4 antibody. The luminescence values of the acridinium esters were measured, and the results of the test are shown in Table 3.

**Table 3**

| Sample No. | Signal value | | |
|---|---|---|---|
| | Example 2 | Example 3 | Example 4 |
| 1 | 3007009 | 2050412 | 2437253 |
| 2 | 501354 | 294123 | 405368 |
| 3 | 302748 | 190069 | 230326 |
| 4 | 243369 | 151456 | 195236 |
| 5 | 20284 | 16856 | 17378 |
| 6 | 205812 | 14753 | 16206 |
| 7 | 170213 | 10325 | 13269 |
| 8 | 90012 | 5321 | 75213 |

As can be seen from Table 3, the signal values were higher for a molar ratio of BSA to ethylenediamine of 1: 10 to 1:20 compared to a molar ratio of BSA to ethylenediamine of 1:5. This shows that by adjusting the molar ratio of cBSA to ethylenediamine, the degree of cationization of BSA can be adjusted, which in turn can improve the signal-to-noise ratio of the final test.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present disclosure.

The above-described embodiments are only several implementations of the present application to facilitate specific and detailed understanding of the technical solutions of the present application, but they should not be construed as limiting the protection scope of the present application. It should be understood by those of ordinary skill in the art that various modifications and improvements can be made without departing from the concept of the present application, and all fall within the protection scope of the present application. It should be understood that technical solutions obtained based on the technical solutions provided in the present application by a person skilled in the art through logical analysis, reasoning, or limited experimentation shall all fall within the protection scope of the appended claims of the present application. Therefore, the patent protection of the present invention shall be defined by the appended claims, and the specification and the drawings can be used to explain the content of the claims.

## Claims

1. A preparation method of a coated magnetic bead, comprising steps of:
modifying a bovine serum albumin with a polyamine to produce a cationized bovine serum albumin; and
coupling an active substance to a magnetic nanobead via the cationized bovine serum albumin to produce the coated magnetic bead.

2. The preparation method of claim 1, **characterized in that** the step of coupling the active substance to the magnetic nanobead via the cationized bovine serum albumin to produce the coated magnetic bead comprising:
coupling the cationized bovine serum albumin to the active substance to produce a complex; and
coupling the complex to the magnetic nanobead to produce the coated magnetic bead.

3. The preparation method of claim 1, **characterized in that** the polyamine is a diamine and/or a PEG-modified diamine.

4. The preparation method of claim 3, **characterized in that** the diamine is selected from at least one of ethylenediamine, 1-3 diaminopropane, diaminodipropylamine, or hexanediamine.

5. The preparation method of claim 3, **characterized in that** the step of modifying the bovine serum albumin with the polyamine to produce the cationized bovine serum albumin comprises:
mixing the polyamine, the bovine serum albumin, and an activator and subjecting them to a dehydration condensation reaction to produce the cationized bovine serum albumin.

6. The preparation method of claim 5, **characterized in that** a molar ratio of the bovine serum albumin to the polyamine is 1: 5 to 1:20.

7. The preparation method of claim 5, **characterized in that** the activator comprises EDC and at least one of sulfo-NHS or NHS.

8. The preparation method of any one of claims 2 to 7, **characterized in that** a ratio of the complex to the magnetic nanobead is 40 µg:1 mg to 80 µg:1 mg.

9. A coated magnetic bead, **characterized by** produced by the preparation method of any one of claims 1 to 8.

10. A detection kit, **characterized by** comprising the coated magnetic bead of claim 9.
